# EUROPEAN PATENT SPECIFICATION

(11) **EP 4 397 649 B1**
(45) Date of publication and mention of the grant of the patent: **29.10.2025**
(21) Application number: 23863413.3
(22) Date of filing: 01.09.2023
(51) Int. Cl.: C07C 29/14, C07C 29/74, C07C 31/20, C07C 29/141

(54) **METHOD FOR PREPARING NEOPENTYL GLYCOL**
VERFAHREN ZUR HERSTELLUNG VON NEOPENTYLGLYKOL
PROCÉDÉ DE PRÉPARATION DE NÉOPENTYLGLYCOL

(30) Priority: 08.09.2022 KR 20220114486; 25.08.2023 KR 20230111976
(43) Date of publication of application: 10.07.2024
(73) Proprietor: LG Chem, Ltd., Yeongdeungpo-gu Seoul 07336 (KR)
(72) Inventor: KIM, Sung Kyun, Daejeon 34122 (KR); LEE, Sung Kyu, Daejeon 34122 (KR); HA, Na Ro, Daejeon 34122 (KR); CHO, Seung Sik, Daejeon 34122 (KR)
(74) Representative: Goddar, Heinz J.
(86) International application number: PCT/KR2023/013050
(87) International publication number: WO 2024/053937

(56) References cited:
- EP-A1- 3 219 698
- CN-A- 113 952 895
- CN-U- 202 186 949
- JP-A- H11 130 727
- KR-A- 20160 133 843
- KR-A- 20170 029 311
- US-A- 4 855 515

## Description

### [Technical Field]

The present invention relates to a method for preparing neopentyl glycol, and more particularly, to a method for stably performing heat control in a hydrogenation reaction.

### [Background Art]

Neopentyl glycol may be generally prepared by performing an aldol condensation reaction of isobutyl aldehyde and formaldehyde in the presence of a catalyst to form hydroxypivaldehyde and proceeding with a hydrogenation reaction of the hydroxypivaldehyde.

However, since the hydrogenation reaction is an exothermic reaction, a temperature inside a hydrogenation reactor where the hydrogenation reaction is performed is excessively increased, and thus, safety problems arose. In addition, a large amount of by-products is produced as a side reaction by a temperature rise, and a conversion rate to neopentyl glycol which is a product to be obtained is decreased.

Therefore, it is a situation in which a process of stably lowering the raised temperature due to the hydrogenation reaction should be introduced. CN202186949U discloses a method for producing neopentyl glycol from hydroxypivalaldehyde. The method comprises use of two hydrogenation reactors.

### [Disclosure]

### [Technical Problem]

In order to solve the problems mentioned in the Background Art, an object of the present invention is to provide a method for preparing neopentyl glycol, which may lower a temperature increased by a hydrogenation reaction more stably while obtaining high-purity neopentyl glycol with a high recovery rate.

### [Technical Solution]

In one general aspect, provided is a method for preparing neopentyl glycol that includes: supplying a feed stream including hydroxypivaldehyde and a hydrogen supply stream to a first hydrogenation reactor and performing a hydrogenation reaction to obtain a first reaction product including neopentyl glycol; supplying the first reaction product to a flash drum to obtain an upper discharge stream including hydrogen and a first degassed solution stream from which hydrogen has been degassed; supplying an upper discharge stream from the flash drum to a second hydrogenation reactor; supplying the first degassed solution stream to a first heat exchanger to lower the temperature; circulating a part of the first degassed solution stream having the lowered temperature to the first hydrogenation reactor as a first branch stream and supplying the rest to the second hydrogenation reactor as a second branch stream; hydrogenating hydroxypivaldehyde included in the second branch stream in the second hydrogenation reactor using hydrogen included in the upper discharge stream from the flash drum to obtain a second reaction product including neopentyl glycol; and introducing the second reaction product to a neopentyl glycol purification process to obtain neopentyl glycol.

Further embodiments are disclosed in the dependent claims.

### [Advantageous Effects]

According to the method for preparing neopentyl glycol of the present invention, a first reaction product from which gaseous hydrogen is separated by a flash drum is supplied to a first heat exchanger, thereby increasing heat exchange efficiency as compared with a case of providing a separate cooler in a front end of the flash drum. As such, gaseous components including gaseous hydrogen are first removed by the flash drum, thereby stably performing temperature control in the first heat exchanger, when a degassed solution from which the gaseous components have been removed is supplied to the first heat exchanger. That is, in lowering the temperature raised by an exothermic reaction in a hydrogenation reaction, the degassed solution from which gaseous hydrogen is first separated is added to the first heat exchanger, thereby improving heat exchange efficiency in the first heat exchanger and performing stable heat control.

Furthermore, waste heat in the first heat exchanger is heat exchanged with a low-temperature steam or heat exchanged with a lower discharge stream from an aldol purification column performed in an aldol purification process, thereby using energy more efficiently.

### [Description of Drawings]

FIG. 1 is a process flow diagram showing a hydrogenation reaction in a method for preparing neopentyl glycol according to an exemplary embodiment of the present invention.
FIG. 2 is a process flow diagram showing a hydrogenation reaction in a method for preparing neopentyl glycol according to the comparative example of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present invention, the term "stream" may refer to a fluid flow in a process or may refer to a fluid itself flowing in a pipe. Specifically, the "stream" may refer to both a fluid itself flowing in a pipe connecting each device and a fluid flow. In addition, the fluid may refer to one or more of gas and liquid, and a case in which a solid component is included in the fluid is not excluded.

Meanwhile, in the devices such as an extraction column, a purification column, a distillation column, a reactor, a flash drum, and a recovery column in the present invention, unless otherwise particularly stated, a "lower portion" of the device refers to a point at a height of 95% to 100% from the top to the bottom of the device, specifically the lowest part (bottom). Likewise, unless otherwise particularly stated, an "upper portion" of the device refers to, a point at a height of 0% to 5% from the top to the bottom of the device, specifically the highest part (top).

Meanwhile, in the devices such as an extraction column, a purification column, a distillation column, and a recovery column in the present invention, unless otherwise particularly stated, an operating temperature of the device may refer to a temperature in the lower portion of the device. Likewise, unless otherwise particularly stated, an operating pressure of the device may refer to a pressure in the upper portion of the device.

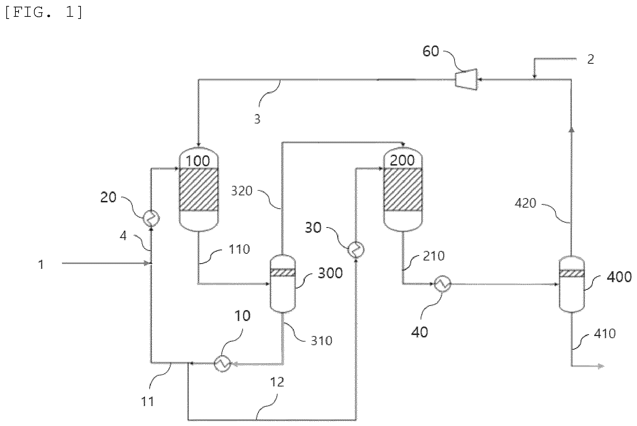

Hereinafter, the present invention will be described in more detail with reference to the FIG. 1 for better understanding of the present invention.

According to an exemplary embodiment of the present invention, provided is a method for preparing neopentyl glycol that includes: supplying a feed stream including hydroxypivaldehyde and a hydrogen supply stream to a first hydrogenation reactor and performing a hydrogenation reaction to obtain a first reaction product including neopentyl glycol; supplying the first reaction product to a flash drum to obtain an upper discharge stream including hydrogen and a first degassed solution stream from which hydrogen has been degassed; supplying an upper discharge stream from the flash drum to a second hydrogenation reactor; supplying the first degassed solution stream to a first heat exchanger to lower the temperature; circulating a part of the first degassed solution stream having the lowered temperature to the first hydrogenation reactor as a first branch stream and supplying the rest to the second hydrogenation reactor as a second branch stream; hydrogenating hydroxypivaldehyde included in the second branch stream in the second hydrogenation reactor using hydrogen included in the upper discharge stream from the flash drum to obtain a second reaction product including neopentyl glycol; and introducing the second reaction product to a neopentyl glycol purification process to obtain neopentyl glycol.

First, the method for preparing neopentyl glycol according to an exemplary embodiment of the present invention may include supplying a feed stream including hydroxypivaldehyde and a hydrogen supply stream including hydrogen to a first hydrogenation reactor and performing a hydrogenation reaction to obtain a first reaction product including neopentyl glycol.

According to an exemplary embodiment of the present invention, the feed stream including hydroxypivaldehyde may be obtained from a feed stream preparation process performed by including an aldol reaction process of obtaining a condensation reaction product by an aldol condensation reaction of a formaldehyde aqueous solution and isobutyl aldehyde in the presence of a catalyst, an aldol extraction process of bringing the condensation reaction product into contact with an extractant to obtain an extract and a raffinate, a saponification reaction process of reducing the raffinate to a catalyst, and an aldol purification process of distilling the catalyst and the extract to separate a fraction including hydroxypivaldehyde as a feed stream.

The feed stream preparation process may be a series of processes performed for preparing a feed stream for being supplied to the first hydrogenation reactor. The feed stream preparation process may be performed by including the aldol reaction process, the aldol extraction process, the saponification reaction process, and the aldol purification process.

Specifically, in the aldol condensation reaction performed in the aldol reaction process, an aldol condensation reaction temperature may be 70°C to 100°C, and an aldol condensation reaction time may be 0.1 hours to 3 hours. In the aldol condensation reaction conditions as such, an aldol condensation reaction of a formaldehyde (FA) aqueous solution and isobutyl aldehyde (IBAL) may be performed in the presence of a catalyst to produce a catalyst salt and hydroxypivaldehyde (HPA).

Herein, formalin may be used as the formaldehyde aqueous solution, in which a concentration of the formalin of 35 to 45 wt% may be effective in terms of wastewater reduction. The formaldehyde aqueous solution may include water in an amount of 40 to 64 wt%, more specifically 45 to 55 wt% with respect to the total weight of the formaldehyde aqueous solution, and may include methanol for preventing polymerization of formaldehyde. In this case, the content of methanol may be 0.1 to 15 wt%, more specifically 0.1 to 5 wt% with respect to the total weight of formaldehyde aqueous solution.

The catalyst may be an amine-based compound. Specifically, a tertiary amine compound such as trialkylamine, trimethylamine, triethylamine, tripropylamine, triisopropylamine, and tributylamine may be appropriate. More specifically, the catalyst may include triethyl amine (TEA). In the present invention, since TEA is most efficient in the aldol condensation reaction, it may be used as the catalyst.

Meanwhile, the catalyst salt may be produced by reacting formic acid produced from a Cannizzaro side reaction occurring in the aldol condensation reaction and the catalyst.

In addition, hydroxypivalic acid-neopentyl glycol ester (HPNE) may be further produced by a Tishchenko reaction which is another side reaction of the aldol condensation reaction. Therefore, a condensation reaction product including the catalyst salt, HPNE, and HPA may be obtained in the aldol reaction process.

Subsequently, an aldol extraction process may be performed by bringing the condensation reaction product into contact with an extractant, and an organic phase extract including the catalyst, unreacted IBAL, the extractant, and HPA and a liquid phase raffinate including the catalyst salt may be obtained. Herein, the unreacted IBAL may be unreacted IBAL which is not aldol condensation reacted in the aldol reaction process. The catalyst salt may be present in a state of being dissociated in water, and the water may be derived from a formic acid aqueous solution.

Herein, the extractant may be aliphatic alcohols, and preferably, may include 2-ethylhexanol (2-EH). Since HPA included in the condensate reaction product is soluble in the 2-EH, it may be preferably used in the aldol extraction process using an extraction device according to a liquid-liquid contact method.

Meanwhile, an operating temperature of one or two or more aldol extraction columns performed in the aldol extraction process may be 40°C to 90°C. By operating the aldol extraction column within the temperature range, it may be easy to separate the phase into an organic phase and a liquid phase.

Meanwhile, the raffinate including the catalyst salt is saponified, thereby performing a saponification reaction process of reducing the catalyst salt to a catalyst. In the saponification reaction process, a saponification reaction by a reaction of a separately added inorganic strong base such as sodium hydroxide (NaOH) and the catalyst salt may be performed. Thus, the catalyst salt may be reduced to the catalyst.

Meanwhile, the aldol purification process may be a process of distilling the reduced catalyst obtained from the saponification reaction process and an extract separated from the aldol extraction process to separate unreacted IBAL and the catalyst, and HPA and the extractant. The separated unreacted IBAL and the catalyst may be circulated to the aldol reaction process and reused as a raw material of the aldol condensation reaction. Meanwhile, the HPA and the extractant separated from unreacted IBAL and the catalyst may be supplied to a first hydrogenation reactor 100 as a feed stream 1 of the present invention.

Specifically, the aldol purification process may be performed by one or two or more aldol purification columns. First, when the aldol purification process is performed by one aldol purification column, the unreacted IBAL and the catalyst may be separated from an upper portion, and the HPA and the extractant may be separated from a lower portion of the one aldol purification column, respectively. Meanwhile, when the aldol purification process is performed in two or more aldol purification columns, it may be performed by one or more aldol purification columns which separate unreacted IBAL to the upper portion and one or more aldol purification columns which separate the catalyst to the upper portion. A stream including HPA or the extractant may be separated to the lower portion of the two or more aldol purification columns and discharged, and it may be supplied to the first hydrogenation reactor 100 as the feed stream 1 of the present invention, as described above.

The stream supplied to the first hydrogenation reactor 100 may be the feed stream 1 and a hydrogen supply stream 3. Herein, the hydrogen supply stream 3 may include compressed hydrogen, and the compressed hydrogen may be compressed hydrogen in which fresh hydrogen added through a hydrogen addition stream 2 is compressed through a compressor 60. In the first hydrogenation reactor 100, a hydrogenation reaction of HPA included in the feed stream 1 and compressed hydrogen included in the hydrogen supply stream 3 proceeds to produce neopentyl glycol (NPG). Herein, the hydrogenation reaction may be performed in the presence of a hydrogenation reaction catalyst.

A copper-based catalyst or a nickel catalyst may be used as the hydrogenation reaction catalyst. An example of the copper-based catalyst may be a CuO/BaO/SiO catalyst, and the CuO/BaO/SiO catalyst may be a catalyst of (CuO)ₓ(BaO)_{y}(SiO)_{z} (x, y, and z are wt%, and x:y:z = 10-50:0-10:40-90, 10-50:1-10:40-89, or 29-50:1-10:40-70). The sum of x and y is preferably 20 to 50 (wt%) or 30 to 50 (wt%), based on the sum of x, y, and z (100 wt%), and in this range, performance of the hydrogenation reaction catalyst is excellent and an effect of long life is shown.

Meanwhile, since the hydrogenation reaction is basically an exothermic reaction, there may be difficulty in controlling the temperature inside the hydrogenation reactor in which a hydrogenation reaction is performed. Therefore, the temperature inside the hydrogenation reactor is excessively raised as the hydrogenation reaction proceeds, and safety problems may arise. In addition, by-products, for example, trimethylpentanediol (2,2,4-trimethyl-1,3-pentanediol, TMPD) are produced by a side reaction due to the temperature rise, so that a conversion rate to NPG to be obtained in the present invention may be lowered. Therefore, heat control to lower the temperature raised by the hydrogenation reaction needs to be performed.

Meanwhile, the operating temperature of the first hydrogenation reactor 100 may be 100°C to 200°C, specifically 130°C to 180°C, or 150°C to 170°C, and the operating pressure may be 20 kg/cm² to 50 kg/cm², specifically 30 kg/cm² to 45 kg/cm², or 35 kg/cm² to 45 kg/cm². By operating the first hydrogenation reactor 100 within the range, a hydrogenation reaction of reacting HPA and hydrogen may be performed well to obtain NPG with a high conversion rate.

By the hydrogenation reaction performed in the first hydrogenation reactor 100, the first reaction product including NPG may be obtained. In addition to this, unreacted hydrogen during the hydrogenation reaction may also be included in the first reaction product. Herein, when a hydrogen fraction in the first hydrogenation reactor 100 is high depending on the operating situation of the process, a hydrogen fraction included in the first reaction product may also be increased, and when the hydrogen fraction in the first reaction product is high as such, hydrogen may be present as gaseous hydrogen as well as liquid hydrogen.

Subsequently, the method for preparing neopentyl glycol according to an exemplary embodiment of the present invention may include supplying the first reaction product to a flash drum 300 as a discharge stream 110 from the first hydrogenation reactor to obtain an upper discharge stream 320 including hydrogen and a first degassed solution stream 310 from which hydrogen has been degassed.

Specifically, the first reaction product is distilled in the flash drum 300, thereby separating an upper fraction of the flash drum including gaseous components and a lower fraction of the flash drum including the first degassed solution. Herein, the gaseous components may specifically include gaseous hydrogen. The first degassed solution may be a remainder after removing the gaseous components from the first reaction product, and specifically, may include liquid hydrogen, unreacted HPA, and NPG.

Herein, an operating temperature of the flash drum 300 may be 100°C to 200°C, specifically 130°C to 180°C, or 150°C to 170°C. An operating pressure of the flash drum 300 may be 20 kg/cm² to 60 kg/cm², specifically 25 kg/cm² to 55 kg/cm², or 20 kg/cm² to 40 kg/cm². The temperature and the pressure of the flash drum 300 are operated within the range, whereby it may be easy to separate gaseous hydrogen in the first reaction product introduced to the flash drum 300.

Meanwhile, the method for preparing neopentyl glycol according to an exemplary embodiment of the present invention may include supplying the upper discharge stream 320 of the flash drum to a second hydrogenation reactor 200 and supplying the first degassed solution stream 310 to the first heat exchanger 10 to lower the temperature.

Specifically, the gaseous upper fraction of the flash drum 300 may be supplied to the second hydrogenation reactor 200 as the upper discharge stream 320 of the flash drum, and the liquid lower fraction of the flash drum 300 may be supplied to the first heat exchanger 10 as the first degassed solution stream 310.

An amount of liquid hydrogen in the first degassed solution stream 310 may be 30 ppm or less, 25 ppm or less, or 20 ppm or less. Hydrogen in the first degassed solution stream 310 is included within the range, whereby it may be easy to control the temperature of the first degassed solution stream 310 supplied to the first heat exchanger 10 described later to be within an appropriate range. Herein, the hydrogen included in the first degassed solution stream 310 may be present in a liquid phase.

According to an exemplary embodiment of the present invention, the first degassed solution stream 310 may be supplied to the first heat exchanger 10 to lower the temperature. Specifically, since the temperature of the first degassed solution stream 310 from which gaseous hydrogen has been removed through the flash drum 300 described above may be lowered in the first heat exchanger 10, heat exchange efficiency in the first heat exchanger 10 may be increased. For example, when a stream having a high hydrogen fraction is directly added to the heat exchanger without passing through the flash drum, a content of lighter components (lights) is high in the stream having a high hydrogen fraction, which may be a cause of a decrease in a logarithmic mean temperature difference (LMTD) of the heat exchanger, resulting in deterioration of performance of the heat exchanger so that heat control may be difficult.

Therefore, since in the first heat exchanger 10, the temperature of the first degassed solution stream 310 from which gaseous hydrogen has been degassed by the flash drum 300 is lowered, heat control may be stably performed. As described above, the heat control needs to be performed to address a safety problem in the process due to an exothermic reaction, a problem of an unstable process flow, and a problem of a low conversion rate to NPG. Herein, the heat control may be performed in the heat exchanger of the present invention, which may be lowering the temperature raised by the exothermic reaction to an optimal temperature range.

Meanwhile, the first degassed solution stream 310 is a stream at a relatively high temperature, and when it is cooled in the first heat exchanger 10, energy may be used more efficiently by reusing waste heat.

According to the present invention, the first heat exchanger 10 may be a steam generator, and heat may be stably recovered by the steam generator. As an example, the first degassed solution stream 310 exchanges heat with the low-temperature steam through the first heat exchanger 10 which is the steam generator, thereby being used in low-pressure steam production. As another example, the first degassed solution stream 310 exchanges heat with the lower discharge stream from the aldol purification column in which an aldol purification process is performed, and a part of the lower discharge stream from the aldol purification column is refluxed to the aldol purification column, thereby being used in raising the temperature of the aldol purification column. In this case, the first heat exchanger 10 may be a reboiler of one or more aldol purification columns in which the aldol purification process is performed.

Meanwhile, when the heat exchanger is not provided in a downstream of the flash drum 300 as in the present invention, but provided in a front end of the flash drum 300, that is, in a downstream of the first hydrogenation reactor 100, in the case in which the hydrogen fraction in the stream supplied to the heat exchanger is high, the temperature in the heat exchanger should be further lowered for a stable process flow, and thus, a cooler may be used as the heat exchanger. In this case, since the cooler is used as the heat exchanger, it may be impossible to reuse waste heat from the cooler.

Meanwhile, the method for preparing neopentyl glycol of the present invention may include circulating a part of the first degassed solution stream having the lowered temperature to the first hydrogenation reactor as a first branch stream and supplying the rest to the second hydrogenation reactor as a second branch stream.

Specifically, a part of the first degassed solution stream 310 of which the temperature is stably lowered in the first heat exchanger 10 is branched into the first branch stream 11 and the rest is branched into the second branch stream 12, and the first branch stream 11 is circulated to the first hydrogenation reactor and the second branch stream 12 may be introduced to the second hydrogenation reactor 200.

Herein, a mass flow rate ratio between a flow rate of the first branch stream 11 and a flow rate of the second branch stream 12 may be 3:1 to 7:1, specifically 4:1 to 6:1. By controlling the mass flow rate ratio to be within the range, two streams branched through the first heat exchanger 10, that is, the first branch stream 11 and the second branch stream 12 may be supplied at an appropriate ratio to the first hydrogenation reactor 100 and the second hydrogenation reactor 200. Thus, the temperature raised by the hydrogenation reaction in the process may be stably lowered in the heat exchanger.

More specifically, when the mass flow rate ratio is less than 3:1, the flow rate of the second branch stream 12 is relatively increased, so that the temperature is excessively raised during the hydrogenation reaction in the second hydrogenation reactor 200 to which the second branch stream 12 is supplied, which may make temperature lowering in a fourth heat exchanger 40 difficult. Meanwhile, the mass flow rate ratio is more than 7:1, the flow rate of the first branch stream 11 is excessively increased, so that energy may be excessively used in separating a gas phase and a liquid phase in the flash drum 300 and lowering the temperature in the first heat exchanger 10, after the hydrogenation reaction in the first hydrogenation reactor 100.

In addition, the temperature of the first branch stream 11 may be 110°C to 140°C. Specifically, the temperature of the first branch stream 11 may be 120°C to 135°C or 125°C to 135°C. The first branch stream 11 is supplied to the first hydrogenation reactor 100 through the second heat exchanger 20 at a temperature within the range, thereby being introduced to the first hydrogenation reactor 100 again in a temperature-lowered state even in the case in which the temperature is raised by the hydrogenation reaction, and thus, the temperature of the first reaction product may not be excessively raised. Therefore, when the temperature is lowered, accidents such as fire and explosion due to a runaway reaction may be prevented from occurring.

In addition, the first branch stream 11 is mixed with the feed stream 1 to form a mixed stream 4, and the mixed stream 4 may be supplied to the first hydrogenation reactor 100 through the second heat exchanger 20. Specifically, the temperature of the mixed stream 4 is lowered again in the second heat exchanger 20, whereby the lower discharge stream 110 from the first hydrogenation reactor may not be discharged at an excessively high temperature due to the hydrogenation reaction performed in the first hydrogenation reactor 100.

Meanwhile, the second branch stream 12 including the first degassed solution stream of which the temperature is lowered by the first heat exchanger 10 may be supplied to the second hydrogenation reactor 200 in a temperature-lowered state again through a third heat exchanger 30.

The method for preparing neopentyl glycol according to an exemplary embodiment of the present invention may include hydrogenating HPA included in the second branch stream 12 in the second hydrogenation reactor 200 using hydrogen included in the upper discharge stream 320 of the flash drum to obtain the second reaction product including NPG. Herein, the second branch stream 12 may further include NPG produced in the first hydrogenation reactor 100.

Meanwhile, the operating temperature of the second hydrogenation reactor 200 may be 100°C to 200°C, specifically 130°C to 180°C, or 150°C to 170°C, and the operating pressure may be 20 kg/cm² to 50 kg/cm², specifically 30 kg/cm² to 45 kg/cm², or 35 kg/cm² to 45 kg/cm². The operating conditions of the second hydrogenation reactor 200 as such may be the same as the operating conditions of the first hydrogenation reactor 100.

Meanwhile, NPG may be further produced by the hydrogenation reaction of HPA and hydrogen also in the second hydrogenation reactor 200, as in the hydrogenation reaction in the first hydrogenation reactor 100 described above. The second reaction product including NPG produced as such may be obtained, and the second reaction product may be supplied to a degassing unit 400 through the fourth heat exchanger 40 as the lower discharge stream 210 of the second hydrogenation reactor. The temperature of the second reaction product is lowered in the fourth heat exchanger 40, thereby lowering pressure in the degassing column of the degassing unit 400 described later to perform degassing better.

According to an exemplary embodiment of the present invention, the second reaction product may be supplied to the degassing unit 400 to introduce the second degassed solution stream 410 from which hydrogen has been degassed to the neopentyl glycol purification process. Herein, the hydrogen may be hydrogen which is not hydrogenated with HPA in the second hydrogenation reactor 200, and the hydrogen may be further degassed in the degassing unit 400. In the degassing unit 400, degassing may be performed to obtain an upper discharge stream 420 from the degassing unit including hydrogen and a second degassed solution stream 410 including NPG. The upper discharge stream 420 from the degassing unit may be supplied to a compressor 60, and the second degassed solution stream 410 may be introduced to the NPG purification process described later.

Herein, the degassing unit 400 may include one or more degassing columns to perform degassing. For example, the second reaction product may be first degassed in the first degassing column, and the stream discharged to the upper portion of the first degassing column may be degassed once more. As such, the stream degassed once more may be supplied to the first hydrogenation reactor 100 through the compressor 60. Further, a part of the stream discharged through the compressor 60 may be branched, temperature-lowered through the heat exchanger, and then circulated to the front end of the compressor 60. Meanwhile, the stream discharged to the lower portion of the first degassing column may also be degassed once more, and the stream from which hydrogen has been degassed as such may be subjected to an NPG purification process.

Subsequently, the method for preparing neopentyl glycol according to an exemplary embodiment may include introducing the second reaction product to the neopentyl glycol purification process to obtain neopentyl glycol.

Specifically, the NPG purification process for obtaining the NPG from the second degassed solution stream 410 including NPG may be performed. In addition to this, the second degassed solution stream 410 may further include HPNE, the catalyst, and the extractant.

The neopentyl glycol purification process of the present invention may be a process of distilling the second degassed solution stream 410 to obtain neopentyl glycol. Specifically, in the NPG purification process, the second degassed solution stream 410 is distilled to be separated into the catalyst, the extractant, HPNE, and NPG, and NPG separated as such may be obtained as an intended product.

Meanwhile, as described above, the HPNE separated in the NPG purification process is a by-product to NPG which is the intended product, and the stream including HPNE separated in the NPG purification process is subjected to the HPNE purification process, thereby further obtaining NPG which is not recovered in the NPG purification process, and also, recovering HPNE which is a high value-added product itself and using it as a product.

As an example, in the HPNE purification process, the stream including HPNE separated in the NPG purification process is distilled to separate HPNE and a part of NPG. That is, since the part of NPG which is not obtained in the NPG purification process and separated may be recovered from the HPNE purification process, an NPG recovery rate may be further increased. In addition, since the HPNE may be used as a raw material in another process, for example, as a main raw material of coating and synthesis of polyester, economic feasibility may be improved in terms of using raw materials by the HPNE purification process according to the present invention.

Meanwhile, according to an exemplary embodiment of the present invention, the catalyst and the extractant separated in the NPG purification process may be subjected to an extractant purification process. A fraction including the catalyst and a fraction including the extractant may be separated by distillation performed in the extractant purification process. Herein, the catalyst may be a small amount of the catalyst which is not separated in the aldol purification process described above. The catalyst is separated in the extractant purification process and circulated to the aldol purification process, thereby recovering the catalyst in the system, for example, TEA.

Meanwhile, the extractant separated in the extractant purification process may be an unreacted extractant in the aldol extraction process, and may be circulated again to the aldol extraction process. Thus, the extractant, for example, 2-EH may be separated, recovered, and reused.

Hereinafter, the present invention will be described in more detail by the examples. However, the following examples are provided for illustrating the present invention.

### Examples

### Example 1

According to the process flow illustrated in FIG. 1, a preparation process of neopentyl glycol (NPG) was simulated, using an Aspen Plus simulator available from Aspen.

A feed stream 1 including hydroxypivaldehyde (HPA) and a hydrogen supply stream 3 including compressed hydrogen were supplied to the first hydrogenation reactor 100, respectively, and were hydrogenated to obtain a first reaction product including neopentyl glycol. At this time, an operating temperature of the first hydrogenation reactor 100 was 160°C and an operating pressure thereof was 40 kg/cm². A hydrogen fraction included in the first reaction product (hydrogen fraction in the first reaction product) with respect to the total weight of the first reaction product was 17 ppm.

The first reaction product was supplied to a flash drum 300 to obtain an upper discharge stream 320 from the flash drum including hydrogen and a first degassed solution stream 310 from which hydrogen had been degassed. At this time, an amount of hydrogen included in the first degassed solution stream 310 (hydrogen fraction of a heat exchanger inlet) was 17 ppm.

Subsequently, the upper discharge stream 320 from the flash drum was supplied to a second hydrogenation reactor 200, and the first degassed solution stream 310 was supplied to a first heat exchanger 10 to lower the temperature to 130°C. The first heat exchanger 10 was a steam generator. A part of the first degassed solution stream having the lowered temperature was circulated to the first hydrogenation reactor 100 as a first branch stream 11, and the rest was supplied to the second hydrogenation reactor 200 as a second branch stream 12.

Specifically, the first branch stream 11 was mixed with the feed stream 1 and formed a mixed stream 4, and the mixed stream 4 was supplied to the first hydrogenation reactor 100 through a second heat exchanger 20. Meanwhile, the second branch stream 12 was supplied to the second hydrogenation reactor 200 in a temperature-lowered state again through a third heat exchanger 30.

In the second hydrogenation reactor 200, HPA included in the second branch stream 12 were hydrogenated using hydrogen included in the upper discharge stream 320 from the flash drum to obtain the second reaction product including neopentyl glycol.

The second reaction product was supplied to a degassing unit 400 through a fourth heat exchanger 40 as the lower discharge stream 210 from the second hydrogenation reactor. At this time, a temperature of a stream which was passed through the fourth heat exchanger 40 was 40°C. In the degassing unit 400, the second degassed solution stream 410 from which hydrogen had been further degassed was introduced to a neopentyl glycol purification process to obtain neopentyl glycol.

### Example 2

In Example 2, reactivity in the first hydrogenation reactor was controlled as compared with Example 1, thereby adjusting the fraction of hydrogen included in the first reaction product (hydrogen fraction in the first reaction product) to 1% as compared with the total weight of the first reaction product. In addition, the amount of hydrogen included in the first degassed solution stream 310 (hydrogen fraction of the heat exchanger inlet) was 17 ppm.

### Comparative Examples

### Comparative Example 1

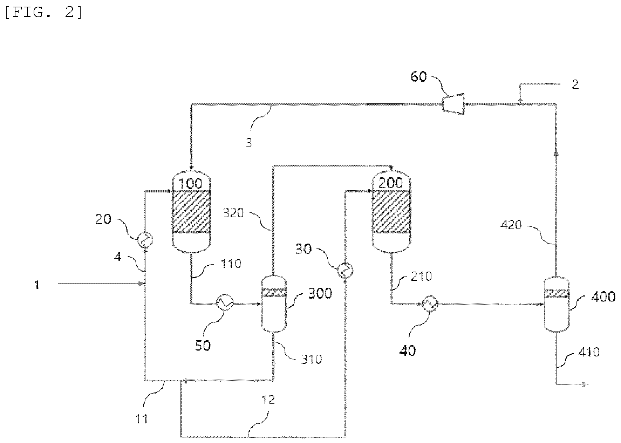

According to the process flow illustrated in FIG. 2, a preparation process of neopentyl glycol (NPG) was simulated, using the Aspen Plus simulator available from Aspen.

In Comparative Example 1, a lower discharge stream 110 from a first hydrogenation reactor including a first reaction product was supplied to a cooler 50 to lower the temperature from 160°C to 130°C, and was supplied to a flash drum 300.

In the flash drum, a first degassed solution stream 310 from which gaseous hydrogen was removed was obtained, and NPG was prepared by the same process flow as in Example 1, except that a mixed stream 4 in which a part of the first degassed solution stream 310 was mixed with a feed stream as a first branch stream 11 was circulated to a first hydrogenation reactor 100 through a heat exchanger 20.

As a result, a hydrogen fraction included in the first reaction product (hydrogen fraction in the first reaction product) with respect to the total weight of the first reaction product was 17 ppm. Since the first reaction product was supplied to the cooler 50, the hydrogen fraction of the heat exchanger (cooler) inlet was also 17 ppm.

### Comparative Example 2

In Comparative Example 2, reactivity in the first hydrogenation reactor was controlled as compared with Comparative Example 1, thereby adjusting the fraction of hydrogen included in the first reaction product (hydrogen fraction in the first reaction product) to 1% as compared with the total weight of the first reaction product. Since the first reaction product was supplied to the cooler 50, the hydrogen fraction of the heat exchanger (cooler) inlet was also 1%.

The following Table 1 shows that the hydrogen fraction in the first reaction product, the hydrogen fraction of the heat exchanger inlet, the vapor fraction of the heat exchanger inlet, the volume increase rate, and the heat recovery amount in the first reaction product of the examples and the comparative examples.

The vapor fraction of the heat exchanger inlet shows a ratio of the mass flow rate of the gaseous components included in the stream supplied to the heat exchanger as compared with the total mass flow rate of the stream supplied to the heat exchanger. Herein, the heat exchanger was the first heat exchanger in the examples, and the cooler in the comparative examples.

**[Table 1]**

| | Hydrogen fraction in first reaction product | Hydrogen fraction in first reaction product | Vapor fraction of heat exchanger¹⁾ inlet | Volume increase ratio | Heat recovery amount |
|---|---|---|---|---|---|
| Example 1 | 17 ppm | 17 ppm | 0 | - | o (5.3754 Gcal/hr) |
| Example 2 | 1% | 17 ppm | 0 | - | o (5.2604 Gcal/hr) |
| Comparative Example 1 | 17 ppm | 17 ppm | 0 | - | × |
| Comparative Example 2 | 1% | 1% | 0.32 | 4.5 | × |
| ¹⁾ The heat exchanger was the first heat exchanger in the examples, and the cooler in the comparative examples. | | | | | |

Referring to Table 1, the examples are the case of supplying the first reaction product to the flash drum to remove gaseous hydrogen and supplying it to the first heat exchanger, and it was confirmed that there was no vapor fraction in the stream added to the first heat exchanger. In addition, it was confirmed in the examples that waste heat was recovered by using the first heat exchanger (steam generator) .

In Example 1, the hydrogen fraction included in the first reaction product was 17 ppm which was a very small amount, and since the hydrogen is present in a liquid phase at this time, it was confirmed that the hydrogen fraction in the stream added to the heat exchanger through the flash drum was the same as the hydrogen fraction included in the first reaction product.

In Example 2, the hydrogen fraction included in the first reaction product was 1%, which was high as compared with Example 1, and when the hydrogen fraction was relatively high as such, both liquid hydrogen and gaseous hydrogen may be present. Therefore, it was confirmed that the first reaction product was supplied to the flash drum to remove gaseous hydrogen, whereby there was no vapor fraction in the stream added to the first heat exchanger. In addition, it was confirmed that hydrogen added to the first heat exchanger was liquid, and the hydrogen fraction in the stream added to the first heat exchanger was 17 ppm.

Meanwhile, in the comparative examples, the first reaction product was first supplied to the cooler to lower the temperature and then was supplied to the flash drum, and in Comparative Example 1, since the hydrogen fraction in the first reaction product was very small as in Example 1, there was no gaseous hydrogen, but waste heat was not able to be recovered by providing the cooler for lowering the temperature of the first reaction product.

In Comparative Example 2, the hydrogen fraction in the first reaction product was high as in Example 2, and both liquid hydrogen and gaseous hydrogen were able to be present together as described above. Since the first reaction product was supplied to the cooler, it was confirmed that the vapor fraction in the stream (first reaction product) added to the cooler was 0.32 and the volume increase fraction was 4.5. The volume increase ratio shows a ratio of a volume of the stream supplied to the heat exchanger (cooler) when the hydrogen fraction of the heat exchanger inlet was 1% (Comparative Example 3) to a volume of the stream supplied to the heat exchanger when the hydrogen fraction of the heat exchanger inlet was 17 ppm (Example 1 and Comparative Example 1). That is, since there was the vapor fraction in the stream supplied to the cooler in Comparative Example 2, gaseous hydrogen was increased, and thus, it was confirmed that the volume of the stream supplied to the cooler was increased as compared with the examples and Comparative Example 1.

## Claims

1. A method for preparing neopentyl glycol, the method comprising:
supplying a feed stream including hydroxypivaldehyde and a hydrogen supply stream to a first hydrogenation reactor and performing a hydrogenation reaction to obtain a first reaction product including neopentyl glycol;
supplying the first reaction product to a flash drum to obtain an upper discharge stream including hydrogen and a first degassed solution stream from which hydrogen has been degassed;
supplying an upper discharge stream from the flash drum to a second hydrogenation reactor;
supplying the first degassed solution stream to a first heat exchanger to lower the temperature;
circulating a part of the first degassed solution stream having the lowered temperature to the first hydrogenation reactor as a first branch stream and supplying the rest to the second hydrogenation reactor as a second branch stream;
hydrogenating hydroxypivaldehyde included in the second branch stream in the second hydrogenation reactor using hydrogen included in the upper discharge stream from the flash drum to obtain a second reaction product including neopentyl glycol; and
introducing the second reaction product to a neopentyl glycol purification process to obtain neopentyl glycol.

2. The method for preparing neopentyl glycol of claim 1, further comprising: supplying the second reaction product to a degassing unit to introduce a second degassed solution stream from which hydrogen has been degassed to the neopentyl glycol purification process.

3. The method for preparing neopentyl glycol of claim 1, wherein the first branch stream is mixed with the feed stream to form a mixed stream, and the mixed stream is supplied to the first hydrogenation reactor through a second heat exchanger.

4. The method for preparing neopentyl glycol of claim 1, wherein a temperature of the first branch stream is 110°C to 140°C.

5. The method for preparing neopentyl glycol of claim 1, wherein the first degassed solution stream exchanges heat with a low-temperature steam through the first heat exchanger or exchanges heat with a lower discharge stream from an aldol purification column.

6. The method for preparing neopentyl glycol of claim 1, wherein a content of liquid hydrogen in the first degassed solution stream is 30 ppm or less.

7. The method for preparing neopentyl glycol of claim 2, wherein a flow rate ratio between a flow rate of the first branch stream and a flow rate of the second branch stream is 3:1 to 7:1.

8. The method for preparing neopentyl glycol of claim 1,
wherein the feed stream including hydroxypivaldehyde is obtained from a feed stream preparation process performed by including:
an aldol reaction process of obtaining a condensation reaction product by an aldol condensation reaction of a formaldehyde aqueous solution and isobutyl aldehyde in the presence of a catalyst,
an aldol extraction process of bringing the condensation reaction product into contact with an extractant to obtain an extract and a raffinate,
a saponification reaction process of reducing the raffinate to a catalyst, and
an aldol purification process of distilling the catalyst and the extract to separate a fraction including hydroxypivaldehyde as a feed stream.

9. The method for preparing neopentyl glycol of claim 8, wherein the catalyst includes triethyl amine (TEA).

10. The method for preparing neopentyl glycol of claim 8, wherein the extractant includes 2-ethyl hexnaol (2-EH).

11. The method for preparing neopentyl glycol of claim 1, wherein the first heat exchanger is a steam generator.

12. The method for preparing neopentyl glycol of claim 2, wherein the neopentyl glycol purification process is a process of distilling the second degassed solution stream to obtain neopentyl glycol.

## Patentansprüche

1. Verfahren zur Herstellung von Neopentylglykol, wobei das Verfahren umfasst:
Zuführen eines Zufuhrstroms, der Hydroxypivaldehyd und einen Wasserstoffzufuhrstrom enthält, zu einem ersten Hydrierungsreaktor und Durchführen einer Hydrierungsreaktion, um ein erstes Reaktionsprodukt zu erhalten, das Neopentylglykol enthält;
Zuführen des ersten Reaktionsprodukts zu einer Entspannungstrommel, um einen oberen Austragsstrom zu erhalten, der Wasserstoff und einen ersten entgasten Lösungsstrom enthält, aus dem Wasserstoff entgast wurde;
Zuführen eines oberen Austragsstroms aus der Entspannungstrommel zu einem zweiten Hydrierungsreaktor;
Zuführen des ersten entgasten Lösungsstroms zu einem ersten Wärmetauscher, um die Temperatur zu senken;
Zirkulieren eines Teils des ersten entgasten Lösungsstroms, der die gesenkte Temperatur aufweist, zu dem ersten Hydrierungsreaktor als einen ersten Zweigstrom und Zuführen des Rests zu dem zweiten Hydrierungsreaktor als einen zweiten Zweigstrom;
Hydrieren von Hydroxypivaldehyd, das in dem zweiten Zweigstrom enthalten ist, in dem zweiten Hydrierungsreaktor unter Verwendung von Wasserstoff, der in dem oberen Austragsstrom aus der Entspannungstrommel enthalten ist, um ein zweites Reaktionsprodukt zu erhalten, das Neopentylglykol enthält; und
Einführen des zweiten Reaktionsprodukts in einen Neopentylglykol-Reinigungsprozess, um Neopentylglykol zu erhalten.

2. Verfahren zur Herstellung von Neopentylglykol nach Anspruch 1, ferner umfassend: Zuführen des zweiten Reaktionsprodukts zu einer Entgasungseinheit, um einen zweiten entgasten Lösungsstrom, aus dem Wasserstoff entgast wurde, in den Neopentylglykol-Reinigungsprozess einzuführen.

3. Verfahren zur Herstellung von Neopentylglykol nach Anspruch 1, wobei der erste Zweigstrom mit dem Zufuhrstrom gemischt wird, um einen gemischten Strom zu bilden, und der gemischte Strom dem ersten Hydrierungsreaktor durch einen zweiten Wärmetauscher zugeführt wird.

4. Verfahren zur Herstellung von Neopentylglykol nach Anspruch 1, wobei eine Temperatur des ersten Zweigstroms 110 °C bis 140 °C beträgt.

5. Verfahren zur Herstellung von Neopentylglykol nach Anspruch 1, wobei der erste entgaste Lösungsstrom Wärme mit einem Niedertemperaturdampf durch den ersten Wärmetauscher austauscht oder Wärme mit einem unteren Austragsstrom aus einer Aldolreinigungssäule austauscht.

6. Verfahren zur Herstellung von Neopentylglykol nach Anspruch 1, wobei ein Gehalt an flüssigem Wasserstoff in dem ersten entgasten Lösungsstrom 30 ppm oder weniger beträgt.

7. Verfahren zur Herstellung von Neopentylglykol nach Anspruch 2, wobei ein Strömungsratenverhältnis zwischen einer Strömungsrate des ersten Zweigstroms und einer Strömungsrate des zweiten Zweigstroms 3:1 bis 7:1 beträgt.

8. Verfahren zur Herstellung von Neopentylglykol nach Anspruch 1,
wobei der Zufuhrstrom, der Hydroxypivaldehyd enthält, aus einem Zufuhrstromherstellungsverfahren erhalten wird, das durchgeführt wird, indem es Folgendes enthält:
einen Aldolreaktionsprozess des Erhaltens eines Kondensationsreaktionsprodukts durch eine Aldolkondensationsreaktion einer wässrigen Formaldehydlösung und von Isobutylaldehyd in Gegenwart eines Katalysators,
einen Aldolextraktionsprozess des Inkontaktbringens des Kondensationsreaktionsprodukts mit einem Extraktionsmittel, um einen Extrakt und ein Raffinat zu erhalten,
einen Verseifungsreaktionsprozess des Reduzierens des Raffinats zu einem Katalysator und
einen Aldolreinigungsprozess des Destillierens des Katalysators und des Extrakts, um eine Fraktion abzutrennen, die Hydroxypivaldehyd als einen Zufuhrstrom enthält.

9. Verfahren zur Herstellung von Neopentylglykol nach Anspruch 8, wobei der Katalysator Triethylamin (TEA) enthält.

10. Verfahren zur Herstellung von Neopentylglykol nach Anspruch 8, wobei das Extraktionsmittel 2-Ethylhexanol (2-EH) enthält.

11. Verfahren zur Herstellung von Neopentylglykol nach Anspruch 1, wobei der erste Wärmetauscher ein Dampferzeuger ist.

12. Verfahren zur Herstellung von Neopentylglykol nach Anspruch 2, wobei der Neopentylglykol-Reinigungsprozess ein Prozess des Destillierens des zweiten entgasten Lösungsstroms ist, um Neopentylglykol zu erhalten.

## Revendications

1. Méthode de préparation de néopentylglycol, la méthode comprenant:
fournir un flux d'alimentation comprenant de l'hydroxypivaldéhyde et un flux d'alimentation en hydrogène à un premier réacteur d'hydrogénation et effectuer une réaction d'hydrogénation pour obtenir un premier produit de réaction comprenant du néopentylglycol;
fournir le premier produit de réaction à un tambour flash afin d'obtenir un flux de décharge haut comprenant de l'hydrogène et un premier flux de solution dégazé dont l'hydrogène a été dégazé ;
fournir un flux de décharge haut provenant du ballon flash à un deuxième réacteur d'hydrogénation;
fournir le premier courant de solution dégazée à un premier échangeur de chaleur afin d'abaisser la température;
faire circuler une partie du premier flux de solution dégazée ayant la température abaissée vers le premier réacteur d'hydrogénation en tant que premier flux de dérivation et fournir le reste au deuxième réacteur d'hydrogénation en tant que deuxième flux de dérivation;
hydrogéner l'hydroxypivaldéhyde contenu dans le deuxième courant de dérivation dans le deuxième réacteur d'hydrogénation à l'aide de l'hydrogène contenu dans le courant de décharge haut provenant du tambour flash afin d'obtenir un deuxième produit de réaction contenant du néopentylglycol; et
introduire le deuxième produit de réaction dans un procédé de purification du néopentylglycol pour obtenir du néopentylglycol.

2. Méthode de préparation de néopentylglycol selon la revendication 1, comprenant en outre: l'alimentation d'une unité de dégazage avec le deuxième produit de réaction afin d'introduire un deuxième flux de solution dégazé dont l'hydrogène a été dégazé dans le procédé de purification du néopentylglycol.

3. Méthode de préparation de néopentylglycol selon la revendication 1, dans laquelle le premier courant de dérivation est mélangé au courant d'alimentation pour former un courant mixte, et le courant mixte est fourni au premier réacteur d'hydrogénation par l'intermédiaire d'un deuxième échangeur de chaleur.

4. Méthode de préparation de néopentylglycol selon la revendication 1, dans laquelle la température du premier courant de dérivation est comprise entre 110 °C et 140 °C.

5. Méthode de préparation du néopentylglycol selon la revendication 1, dans laquelle le premier courant de solution dégazé échange de la chaleur avec une vapeur à basse température à travers le premier échangeur de chaleur ou échange de la chaleur avec un courant de décharge bas provenant d'une colonne de purification aldolique.

6. Méthode de préparation du néopentylglycol selon la revendication 1, dans laquelle la teneur en hydrogène liquide dans le premier courant de solution dégazé est inférieure ou égale à 30 ppm.

7. Méthode de préparation de néopentylglycol selon la revendication 2, dans laquelle le rapport entre le débit du premier courant de dérivation et le débit du deuxième courant de dérivation est compris entre 3:1 et 7:1.

8. Méthode de préparation du néopentylglycol selon la revendication 1,
dans laquelle le flux d'alimentation comprenant de l'hydroxypivaldéhyde est obtenu à partir d'un processus de préparation du flux d'alimentation réalisé en comprenant:
un procédé de réaction aldolique consistant à obtenir un produit de réaction de condensation par une réaction de condensation aldolique d'une solution aqueuse de formaldéhyde et d'isobutylaldéhyde en présence d'un catalyseur,
un procédé d'extraction aldolique consistant à mettre le produit de la réaction de condensation en contact avec un agent d'extraction pour obtenir un extrait et un raffinat,
un procédé de réaction de saponification pour réduire le raffinat à un catalyseur, et
un procédé de purification aldolique consistant à distiller le catalyseur et l'extrait pour séparer une fraction comprenant de l'hydroxypivaldéhyde en tant que flux d'alimentation.

9. Méthode de préparation du néopentylglycol selon la revendication 8, dans lequel le catalyseur comprend de la triéthylamine (TEA).

10. Méthode de préparation du néopentylglycol selon la revendication 8, dans laquelle l'agent d'extraction comprend du 2-éthylhexanol (2-EH).

11. Méthode de préparation du néopentylglycol selon la revendication 1, dans laquelle le premier échangeur de chaleur est un générateur de vapeur.

12. Méthode de préparation du néopentylglycol selon la revendication 2, dans laquelle le processus de purification du néopentylglycol est un processus de distillation du deuxième flux de solution dégazé pour obtenir du néopentylglycol.
